# EUROPEAN PATENT SPECIFICATION

(11) **EP 1 455 602 B1**
(45) Date of publication and mention of the grant of the patent: **08.03.2006**
(21) Application number: 02792103.0
(22) Date of filing: 20.12.2002
(51) Int. Cl.: A23L 1/305, A23C 9/13, A23C 19/09, A23L 1/314

(54) **MODIFIED METHIONINE RICH FOOD PRODUCTS AND PROCESS FOR THEIR MANUFACTURE**
MODIFIERTE METHIONINEREICHE NAHRUNGSMITTEL UND VERFAHREN ZU DEREN HERSTELLUNG
PRODUITS ALIMENTAIRES RICHES EN METHIONINE ET PROCEDE DE FABRICATION CORRESPONDANT

(30) Priority: 21.12.2001 EP 01205069
(43) Date of publication of application: 15.09.2004
(73) Proprietor: DSM IP Assets B.V., 6411 TE Heerlen (NL)
(72) Inventor: VERHOEF, Petra, NL-6708 SN Wageningen (NL); VAN DER MEER, Roelof, NL-6712 HA Ede (NL)
(74) Representative: Matulewicz, Emil Rudolf Antonius
(86) International application number: PCT/NL2002/000854
(87) International publication number: WO 2003/055335

(56) References cited:
- EP-A- 0 034 034
- EP-A- 0 149 829
- WO-A-01/00047
- WO-A-97/34497
- WO-A-99/13737
- FINKELSTEIN J D ET AL: "Effect of dietary cystine on methionine metabolism in rat liver." THE JOURNAL OF NUTRITION. UNITED STATES JUN 1986, vol. 116, no. 6, June 1986 (1986-06), pages 985-990, XP001074061 ISSN: 0022-3166

## Description

### FIELD OF THE INVENTION

The present invention is concerned with methionine rich food products, e.g. food products comprising significant amounts of animal derived protein or proteinaceous material derived from seeds, nuts, grain or pulses, which products have been modified so as to reduce their boosting effect on serum homocysteine concentration. More particularly, the invention is concerned with food products comprising at least 5% proteinaceous matter by weight of dry matter, wherein at least 50% of the proteinaceous matter is of animal origin or derived from seeds, nuts, grain or pulses, and wherein the amino acid profile of the food product has been altered to contain elevated amounts of cysteine, and optionally serine.

Another aspect of the invention is concerned with a method of modifying a methionine rich food product by incorporating therein a source of cysteine, and optionally a source of serine.

### BACKGROUND OF THE INVENTION

Homocysteine (HS-CH₂-CH(NH₂)-COOH) is a substance which has attracted significant scientific interest because of its association with the development of cardiovascular diseases. Numerous scientific studies have shown that patients suffering from myocardial infarction, stroke, or peripheral occlusive arterial disease, exhibit elevated serum homocysteine concentration (hyperhomocysteinemia). A high concentration of homocysteine is regarded a risk factor for cardiovascular disease. Hyperhomocysteinemia has also been associated with other health risks, e.g. manic depressions, seizure disorders, depression, asthma and migraine headaches.

Fasting plasma homocysteine levels have been classified as either, normal (≤15 µmol/L), or one of the following levels of hyperhomocysteinemia; moderate (15-30 µmol/L), intermediate (31-100 µmol/L), or severe (>100 µmol/L). Like blood cholesterol, the relationship of plasma homocysteine to cardiovacular diseases appears to be graded. This means that any increase in plasma homocysteine above normal is associated with an increased risk, and that the higher the elevation, the higher the risk. The mechanistic details of how deviations in plasma homocysteine promote cardiovascular diseases is not completely understood.

It has been established that, following ingestion, methionine rich food products may confer a methionine load which will subsequently cause a substantial increase in plasma homocysteine concentration. Consequently, individuals who are at risk of cardiovascular diseases, would be well advised not to consume methionine rich food products, e.g. dairy and meat products.

The main metabolic pathways for reducing plasma homocysteine levels are:
a. remethylation back to methionine (main co-factors: vitamin B12, folic acid and betaine); and
b. transsulfuration to cysteine via cystathione (main co-factor: vitamin B6).

Proposals have been made in the prior art to administer the aforementioned co-factors to treat hyperhomocysteinemia.

WO 97/34497 (Shapira) describes dairy products having a modified methionine : vitamin B6 ratio (mg/mg) comprising (a) dairy products having a base methionine : vitamin B6 ratio and (b) at least one source of vitamin B6 present in an amount sufficient to modify the base methionine : vitamin B6 ratio to a reduced preselected ratio. In the PCT-application it is observed that it has been desirable to produce dairy products in which the amount of the cofactors of the methionine metabolism, in particular of vitamin B6, and optionally of magnesium, folic acid, vitamin B12, and cysteine are increased.

US 5,795,873 (Allen) describes a method of treating or preventing elevated serum metabolite levels of homocysteine, comprising periodically administering orally a single formulation having between 0.3-10 mg vitamin B12 and 0.1-0.4 mg folic acid. The option of co-administering vitamin B6 is also mentioned.

Wilkcken et al. "Homocystinuria due to cystathione beta-synthase deficiency", Metabolism, 34, 1985(12), pp. 1115-1121 describe that patients suffering from homocystinuria due to cystathione beta-synthase deficiency may successfully be treated by administering betaine. After betaine administration homocysteine level was reduced to near normal, whereas there was a trend towards increased methionine, serine and cysteine concentrations.

Although the administration of vitamin B6, B12, betaine and folic acid (vitamin B11) may suitably be used in the treatment of hyperhomocysteinemia, such treatments are often insufficient to restore serum homocysteine levels to normal levels. Hence, individuals suffering from hyperhomocysteinemia are well advised to follow a strict diet in which they are not allowed to consume high methionine food products. Since dairy products and meat products are generally regarded as methionine rich food products, such a diet prevents the individual from consuming palatable and nutritionally important foodstuffs.

### SUMMARY OF THE INVENTION

Surprisingly it was found that hyperhomocysteinemia may be treated and/or prevented without the need for a diet that precludes the consumption of methionine rich food products. It was discovered that the amino acid profile of methionine rich food products may be altered in such a way that the high levels of methionine contained in such products will not automatically lead to significantly enhanced serum homocysteine levels. In particular, it was unexpectedly established that cysteine has an antagonistic effect on the serum homocysteine elevating effect of methionine, particularly when used in combination with serine.

Although applicants do not wish to be bound by theory it is believed that both cysteine and serine are involved in the metabolisation of homocysteine. It would appear that the ratio in which cysteine and methionine are present in an ingested food product determines the extent to which the ingested methionine will give rise to elevated serum homocysteine concentrations. Thus, even in case of food products containing high levels of methionine, the undesired effects on serum homocysteine levels may be suppressed by adjusting the amino acid balance and particularly the ratio of methionine versus cysteine and optionally the ratio of methionine versus serine.

The benefits of the present invention are particularly appreciated in food products which contain significant amounts of proteinaceous material of animal origin, notably milk, meat, eggs, fish and shellfish, or proteinaceous material derived from seeds, nuts, grain or pulses. The proteinaceous material found in these foodstuffs and food components usually contains relatively high levels of methionine calculated on total amino acids.

It was found that the detrimental effect of methionine rich food products on serum homocysteine level may be significantly reduced provided the amino acid profile of the food product is modified in such a way that the molar ratio of cysteine to methionine is increased to at least 2:1.

### DETAILED DESCRIPTION OF THE INVENTION

Accordingly one aspect of the invention is concerned with a methionine rich food product, i.e. a food product comprising at least 5 % proteinaceous matter by weight of dry matter, wherein at least 50 wt.% of the proteinaceous matter is of animal origin and/or derived from seeds, nuts, grain or pulses, said food product containing the amino acids cysteine [Cys], methionine [Meth] in a molar ratio which meets the following equation: [Cys] / [Meth] ≥ 2:1.

Unless otherwise indicated, whenever reference is made to an amino acid, e.g. methionine or [Meth], said reference is meant to include corresponding amino acid residues. Examples of sources of amino acid residues are proteins, oligopeptides, dipeptides, glycoproteins, lipoproteins etc. For the purpose of this application cystine is considered to comprise 2 cysteine residues. The term "proteinaceous matter" encompasses amino acids, (oligo)peptides and proteins, as well as any other substances that comprise amino acids, peptides (e.g. glycopeptides) and/or proteins (e.g. lipoproteins).

Examples of proteinaceous matter of animal origin include milk protein, meat protein, egg protein, and collagen, as well fractions thereof, e.g. casein, whey protein, meat hydrolysate etc. The term "animal" as used in here not only encompasses vertebrates, but also birds, fish and shellfish. Also in meat obtained from fish and birds high levels of methionine are found.

The detrimental effect of a methionine rich food product on serum homocysteine levels may be suppressed in a particularly effective manner, if cysteine and methionine are present in a molar ratio of at least 3:1.

In a very preferred embodiment of the invention, in addition to cysteine, a significant amount of serine is incorporated in the present foodstuff so as to alter the amino acid profile in such a way that a molar ratio is obtained which meets the following equation: [Ser] /[Meth] ≥ 4:1. Even more preferably, the molar ratio [Ser] / [Meth] is at least 6:1, most preferably at least 8:1.

The benefits of the present invention are most pronounced in food products containing methionine in relatively high amounts, calculated on the total amount of amino acids present therein. Consequently, in a preferred embodiment, the present food product contains methionine in a concentration which exceeds 1 mol.%, even more preferably exceeds 2 mol.%, calculated on the total amount of amino acids contained in the product.

In another preferred embodiment the food product comprises at least 10%, more preferably at least 20% proteinaceous material by weight of dry matter. Preferably the proteinaceous material is of animal origin. The proteinaceous material of animal origin is suitably selected from the group consisting of milk, meat, egg, fish, shellfish, collagen, skin as well as food materials derived therefrom. Preferably the animal source of the proteinaceous material is cattle, pig, sheep, goat, poultry or fish.

The ratio's in which methionine, cysteine and serine are represented in the food product according to the present invention may vary considerably, provided the combined level of cysteine and serine is sufficiently high to compensate for the homocysteine elevating effect of methionine which is present in the same product. In a particularly preferred embodiment, the present food product contains cysteine in an amount relative to its methionine content in accordance with the following equation: [Cys]/[Meth] ≥4:1. Similarly, the minimum amount of serine present in the food product according to the invention is most preferably described by the following equationL [Ser]/[Meth] ≥10:1.

Although, in principle, it is possible to counteract the homocysteine elevating effect of methionine by incorporating only cysteine, it is preferred to employ a combination of these cysteine and serine. Hence, the present food product preferably contains cysteine and serine in a ratio which is described by the following equation: 0.05 ≤ [Cys]/[Ser] ≤2. In a particularly preferred embodiment of the invention, the food product contains cysteine and serine in the following ratio: 0.1 ≤ [Cys]/[Ser] ≤1.

The benefits of the present invention may become manifest in any food products that contain proteinaceous matter with relatively high levels of methionine. It will be understood, however, that in particular food products containing significant amounts of protein may benefit from the present invention. Hence in a preferred embodiment, the present food product comprises at least 10 wt.%, preferably at least 20 wt.% proteinaceous matter.

Examples of food products containing significant amount of proteinaceous matter which are rich in methionine are:
- dairy products such as milk (from e.g. cow or goat), cheese, processed cheese, dairy desserts,
- meat products such as meats (from e.g. cattle, pig, sheep, goat, poultry, fish etc.), processed meat products (e.g. sausages),
- spreads,
- meal replacers (such as bars and shakes),
- nutritional beverages, ice cream,
- soups and sauces,
- dietary and clinical nutritional formulas.

It can be advantageous to additionally incorporate into the present food product substances which act as co-factors in the metabolisation of serum homocysteine. Consequently in a preferred embodiment, the food product contains at least 10 mg/kg vitamin B6 or a precursor thereof. The term "precursor" as used throughout this document, encompasses any substances which, following ingestion of the food product, are capable of liberating the active component (vitamin B6 or a physiologically active metabolite thereof) in the gastointestinal tract or after absorption into the body, e.g. as a result of metabolic breakdown in the liver.

Betaine is another co-factor of homocysteine metabolism, which may advantageously be included in the present food product. Preferably the food product contains betaine and methionine [Meth] in a molar ratio which meets the following equation: [Bet]/[Meth] ≥1: 1.

Since the present food products may suitably be used as (part of) a treatment or prophylactic treatment of hyperhomocysteinemia, another aspect of the invention is concerned with a method of (prophylactically) treating individuals suffering from elevated serum homocysteine levels. Consequently, the invention also relates to food products, as described herein before, for use in a method of treating or preventing elevated plasma concentrations of homocysteine.

Yet another aspect of the present invention relates to a method of modifying a methionine rich food product which contains methionine and optionally cysteine in a molar ratio which meets the following equation: [Cys]/[Meth] ≤1:1, wherein the method comprises the incorporation of a source of cysteine in an amount sufficient to increase said ratio to more than 2:1, preferably to more than 4:1.

In a particularly preferred embodiment of the present method the food product optionally contains serine in a molar ratio which meets the following equation: [Ser]/[Meth] ≤ 3:1, wherein the method comprises the incorporation of a source of serine in an amount sufficient to increase said ratio to more than 4:1, preferably to more than 6:1.

The term "source of cysteine and/or serine" should be understood to include materials which contain cysteine and/or serine in the form of free amino acids and/or amino acid residues.

The source of cysteine and the source of serine may be any foodgrade material that contains relatively high levels of cysteine and/or serine in comparison to methionine. Preferably the source of cysteine contains cysteine and optionally methionine in the following molar ratio: [Cys]/[Meth] ≥ 5:1. More preferably said ratio is at least 8:1. Similarly the source of serine preferably contains serine and optionally methionine in the following molar ratio: [Ser]/[Meth] ≥ 8:1. More preferably the latter ratio is at least 12:1.

It is advantageous to use a source of cysteine or serine that contains a relatively high amount of proteinaceous matter. Thus only a limited amount needs to be added to obtain the right balance between on the one hand methionine and on the other hand cysteine, and optionally serine. In a particularly preferred embodiment of the present method the source of cysteine as well as the source of serine is a protein concentrate comprising at least 40%, preferably at least 60% proteinaceous matter by weight of dry matter. The source of cysteine preferably contains cysteine in an amount of at least 5%, preferably at least 8% by weight of the proteinaceous matter. The source of serine preferably contains serine in an amount of at least 8%, preferably at least 12% by weight of the proteinaceous matter. Preferably the source of cysteine and the source of serine are incorporated in an amount which does not exceed 25 wt.% of the original food product. More preferably said amount does not exceed 10 wt.%.

In order to facilitate absorption of cysteine and serine, it was found to be advantageous to introduce these amino acids into the food product in the form of free amino acids or relatively small peptides. Hydrolysis may advantageously be used to produce free amino acids or small peptides which may subsequently be fractionated so as to yield a fraction which is in enriched in cyteine and/or serine relative to methionine. Thus, in a preferred embodiment of the present method the proteinaceous matter contained by the source of cysteine and the source of serine has a degree of hydrolysis of at least 50%, more preferably a degree of hydrolysis of at least 80%. The sources of cysteine and serine may suitably have been hydrolysed to a sufficient degree by means of enzymatic hydrolysis.

It is well known in the flavour industry to produce reaction flavours (or processed flavours) by heating a mixture of e.g. cysteine and a reducing sugar under conditions that favour so called Maillard reactions. Such reaction flavours are particularly useful for imparting meaty and nutty flavour notes to foodstuffs. According to a preferred embodiment the present invention does not comprise the inclusion of such a reaction flavour, since the reactions occurring during the heating process converts cysteine into components that do not have an antagonistic effect on the serum homocysteine elevating effect of methionine

The present method may advantageously be employed in the manufacture of dairy and meat derived foodstuffs. In particular in case of dairy products, it is often deemed undesirable to 'contaminate' such products with ingredients of non-dairy origin. Hence the source of cysteine and/or serine used in the present method preferably is of the same animal origin as the bulk of the food product. Consequently, in a preferred embodiment of the present method, prior to modification, the food product comprises at least 5 % proteinaceous matter by weight of dry matter, at least 50 wt.% of the proteinaceous matter being of animal origin, and wherein the source of cysteine and/or serine is of the same animal origin as said proteinaceous matter.

Examples of suitable sources of cysteine and/or serine include whey (particularly lactalbumin), protein or protein fractions isolated from pulses and wheat proteins (especially albumine and globuline). In order to further increase the levels of cysteine and/or serine, in favour of methionine, it can be advantageous to subject the aforementioned source materials to a hydrolysation step, followed by the application of an amino acid and/or peptide isolation technique. Such isolation techniques are well known in the art and include, for instance, ion exchange, gel permation and reversed phase chromatogaphy, as well as ultrafiltration, nanoflitration and precipitation.

Yet another aspect of the present invention relates to the use of the combination of cysteine and serine in a product selected from the group of foodstuffs, nutritional products and pharmaceutical preparations, for lowering the serum homocysteine elevating effect of the product and/or for increasing the serum homocysteine lowering effect of the product.

Finally the present invention also encompasses a method of treating or preventing hyperhomocysteinemia in humans by orally administering an effective amount of a food product as defined herein before.

The invention is further illustrated by means of the following examples.

### EXAMPLES

### Example 1

To an ordinary retail yogurt product, obtained from a Dutch dairy, methionine, cysteine (added in the form of cystine) and/or serine are added in the following amounts:

| | Amount in 200 ml yoghurt (without addition) | Composition | | | |
|---|---|---|---|---|---|
| | | Control | I | II | III |
| | | Added amount amino acid per 200 ml yoghurt | | | |
| Methionine | 1.10 mmol | 8.04 mmol | 8.04 mmol | 8.04 mmol | 8.04 mmol |
| | 0.164 g | 1.2 g | 1.2 g | 1.2 g | 1.2 g |
| Cysteine | 0.52 mmol | 0 | 44.6 mmol | 0 | 44.6 mmol |
| | 0.063 g | | 5.4 g | | 5.4 g |
| Serine | 4.30 mmol | | | 57.1 mmol | 57.1 mmol |
| | 0.452 g | 0 | 0 | 6.0 g | 6.0 g |

In a cross-over study 24 subjects ingest a single dose of one of the 4 compositions described in the above table (control, composition I, composition II, composition III) together with a low protein breakfast. The 4 different compositions are ingested by each subject in random order on days 2, 9, 16 and 23.

Blood serum homocysteine levels are measured 0, 2, 4, 6, 8, 10 and 24 hours after breakfast. Homocysteine is found to increase considerably after the subjects have ingested the control composition. The observed increase in homocysteine level is significantly lower after subjects have ingested composition I or II than after the control composition. The increase in homocysteine level is found to be minimal after subjects have ingested composition III.

### Example 2

To the yogurt product described in Example 1 the amino acids cysteine (in the form of cystine) and serine were added in the following amounts:

| | Composition | | | |
|---|---|---|---|---|
| | Control | I | II | III |
| | Amount in 200 ml yoghurt (without addition) | Added amount (mg/200 ml) | | |
| Methionine | 1.10 mmol | 0 | 0 | 0 |
| | 0.164 g | | | |
| Cysteine | 0.52 mmol | 4.87 mmol | 0 | 4.87 mmol |
| | 0.063 g | 0.59 g | | 0.59 g |
| Serine | 4.30 mmol | 0 | 7.69 mmol | 7.69 mmol |
| | 0.452 g | | 0.81 g | 0.81g |

### Example 3

To a beef hamburger the amino acids cysteine (in the form of cystine) and serine were added in the following amounts:

| | Composition | | | |
|---|---|---|---|---|
| | Control | I | II | III |
| | Amount in 100g hanburger (prepared) | Added amount (mg/100g) | | |
| Methionine | 2.73 mmol | 0 | 0 | 0 |
| | 0.408 g | | | |
| Cysteine | 1.58 mmol | 11.80 mmol | 0 | 11.80 mmol |
| | 0.192 g | 1.43 g | | 1.43 g |
| Serine | 6.39 mmol | 0 | 23.37 mmol | 23.37 mmol |
| | 0.672 g | | 2.46 g | 2.46 g |

## Claims

1. Food product comprising at least 5 % proteinaceous matter by weight of dry matter, wherein at least 50 wt.% of the proteinaceous matter is of animal origin and/or derived from seeds, nuts, grain or pulses, said food product
a. containing the amino acids cysteine [Cys] and methionine [Meth] in a molar ratio which meets the following equation: [Cys] / [Meth] ≥ 3:1 ; or
b. containing the amino acids cysteine [Cys] and methionine [Meth] in a molar ratio which meets the following equation: [Cys] / [Meth] ≥ 2:1 and additionally containing serine [Ser] in a molar ratio which meets the following equation: [Ser] / [Meth] > 4:1.

2. Food product according to claim 1 wherein the food product additionally contains serine [Ser] in a molar ratio which meets the following equation: [Ser]/[Meth] ≥ 6:1.

3. Food product according to claim 1 or 2, wherein [Meth] exceeds 1 mol.%, calculated on the total amount of amino acids contained in the product.

4. Food product according to any one of claims 1-3, wherein the food product comprises at least 10%, preferably at least 20% proteinaceous material by weight of dry matter.

5. Food product according to any one of claims 1-4, wherein the food product is selected from the group consisting of dairy products,meat products, spreads, meal replacers, nutritional beverages, ice cream, soups and sauces, dietary and clinical nutritional formulas.

6. Food product according to any one of claims 1-5, wherein the food product contains at least 10 mg/kg vitamin B6.

7. Food product according to any one of claims 1-6, wherein the food product contains betaine [Bet] and methionine [Meth] in a molar ratio which meets the following equation: [Bet]/[Meth] ≥ 1:1.

8. Food product according to any one of claims 1-7, for use in a method of treating or preventing elevated plasma concentrations of homocysteine.

9. Method of modifying a methionine rich food product which contains methionine and optionally cysteine in a molar ratio which meets the following equation: [Cys]/[Meth]≤ 1: 1, wherein the method comprises the incorporation of a source of cysteine in an amount sufficient to increase said ratio to more than 2:1, preferably to more than 4:1.

10. Method according to claim 9, wherein the food product additionally contains serine in a molar ratio which meets the following equation: [Ser]/[Meth] ≤ 3:1, wherein the method comprises the incorporation of a source of serine in an amount sufficient to increase said ratio to more than 4:1, preferably to more than 6:1.

11. Method according to claim 10 or 9, wherein the source of cysteine contains cysteine and optionally methionine in the following molar ratio: [Cys]/[Meth] ≥ 5:1.

12. Method according to any one of claims 9-11, wherein the source of serine contains serine and optionally methionine in the following molar ratio: [Ser]/[Meth] ≥ 8:1.

13. Use of the combination of cysteine and serine in the manufacture of a product for use in the treatment or prevention of hyperhomocysteinemia, said product being selected from the group of foodstuffs, nutritional products and pharmaceutical preparations,

## Patentansprüche

1. Nahrungsmittelprodukt, enthaltend mindestens 5 % Proteinsubstanz, bezogen auf das Gewicht der Trockensubstanz, wobei mindestens 50 Gew.% der Proteinsubstanz tierischen Ursprungs sind und/oder aus Samen, Nüssen, Getreide oder Hülsenfrüchten stammen und wobei das Nahrungsmittelprodukt
a. die Aminosäuren Cystein [Cys] und Methionin [Meth] in einem Molverhältnis enthält, das die folgende Gleichung [Cys] / [Meth] ≥ 3:1 erfüllt, oder
b. die Aminosäuren Cystein [Cys] und Methionin [Meth] in einem Molverhältnis, das die folgende Gleichung [Cys]/[Meth] ≥ 2 : 1 erfüllt, und zusätzlich Serin [Ser] in einem Molverhältnis, das die folgende Gleichung [Ser]/[Meth] ≥ 4:1 erfüllt, enthält.

2. Nahrungsmittelprodukt nach Anspruch 1, worin das Nahrungsmittelprodukt zusätzlich Serin [Ser] in einem Molverhältnis enthält, das die folgende Gleichung [Ser]/Meth] ≥6:1 erfüllt.

3. Nahrungsmittelprodukt nach Anspruch 1 oder 2, worin [Meth] 1 Mol% überschreitet, berechnet aufgrund der Gesamtmenge der in dem Produkt enthaltenen Aminosäuren.

4. Nahrungsmittelprodukt nach einem der Ansprüche 1 bis 3, wobei das Nahrungsmittelprodukt mindestens 10 %, vorzugsweise mindestens 20 %, Proteinsubstanz, bezogen auf das Gewicht der Trockensubstanz, enthält.

5. Nahrungsmittelprodukt nach einem der Ansprüche 1 bis 4, wobei das Nahrungsmittelprodukt aus der Gruppe der Milchprodukte, Fleischprodukte, Brotaufstriche, Mehlersatzstoffe, Nährgetränke, Eiscreme, Suppen, Soßen sowie diätetischen und klinischen Nährstoffformulierungen ausgewählt ist.

6. Nahrungsmittelprodukt nach einem der Ansprüche 1 bis 5, wobei das Nahrungsmittelprodukt mindestens 10 mg/kg Vitamin B6 enthält.

7. Nahrungsmittelprodukt nach einem der Ansprüche 1 bis 6, wobei das Nahrungsmittelprodukt Betain [Bet] und Methionin [Meth] in einem Molverhältnis enthält, das die folgende Gleichung [Bet]/[Meth] ≥ 1:1 erfüllt.

8. Nahrungsmittelprodukt nach einem der Ansprüche 1 bis 7 für die Verwendung in einem Verfahren zum Behandeln oder Verhindern erhöhter Plasmakonzentrationen von Homocystein.

9. Verfahren zum Modifizieren eines an Methionin reichen Nahrungsmittels, das Methionin und gegebenenfalls Cystein in einem Molverhältnis enthält, das die folgende Gleichung [Cys]/[Meth] ≤ 1:1 erfüllt, wobei das Verfahren das Einbringen einer Cysteinquelle in einer Menge beinhaltet, die ausreicht, um das genannte Verhältnis auf über 2:1, vorzugsweise auf über 4:1, zu erhöhen.

10. Verfahren nach Anspruch 9, worin das Nahrungsmittelprodukt zusätzlich Serin in einem Molverhältnis enthält, das die folgende Gleichung [Ser]/[Meth] ≤ 3:1 erfüllt, wobei das Verfahren das Einbringen einer Serinquelle in einer Menge beinhaltet, die ausreicht, um das genannte Verhältnis auf über 4:1, vorzugsweise auf über 6:1, zu erhöhen.

11. Verfahren nach Anspruch 10 oder 9, worin die Cysteinquelle Cystein und gegebenenfalls Methionin im folgenden Molverhältnis [Cys]/[Meth] ≥ 5:1 enthält.

12. Verfahren nach einem der Ansprüche 9 bis 11, worin die Serinquelle Serin und gegebenenfalls Methionin im folgenden Molverhältnis von [Ser]/[Meth] ≥ 8:1 enthält.

13. Verwendung einer Kombination aus Cystein und Serin bei der Herstellung eines Produkts für die Verwendung zur Behandlung oder Verhinderung von Hyperhomocysteinemie, wobei das genannte Produkt aus der Gruppe der Nahrungsmittel, Nährstoffprodukte und pharmazeutischen Zubereitungen ausgewählt ist.

## Revendications

1. Produit alimentaire comprenant au moins 5 % de matière protéique en poids de la matière sèche, dans lequel au moins 50 % en poids de la matière protéique est d'origine animale et/ou dérivée de graines, de noix, de céréales ou de légumineuses, ledit produit alimentaire :
a. contenant les acides aminés cystéine [Cys] et méthionine [Meth] dans un rapport molaire qui satisfait l'équation suivante : [Cys]/[Meth] ≥ 3 :1, ou
b. contenant les acides aminés cystéine [Cys] et méthionine [Meth] dans un rapport molaire qui satisfait l'équation suivante : [Cys]/[Meth] ≥ 2:1, et contenant de plus de la sérine [Ser] dans un rapport molaire qui satisfait l'équation suivante : [Ser]/[Meth] ≥ 4 :1.

2. Produit alimentaire selon la revendication 1, dans lequel le produit alimentaire contient de plus de la sérine [Ser] dans un rapport molaire qui satisfait l'équation suivante : [Ser]/[Meth] ≥ 6 :1

3. Produit alimentaire selon la revendication 1 ou 2, dans lequel la [Meth] dépasse 1 % en mole, calculé sur la quantité totale d'acides aminés dans le produit.

4. Produit alimentaire selon l'une quelconque des revendications 1-3, dans lequel le produit alimentaire comprend au moins 10 %, de préférence au moins 20 % de matière protéique en poids de la matière sèche.

5. Produit alimentaire selon l'une quelconque des revendications 1-4, dans lequel le produit alimentaire est sélectionné dans le groupe constitué des produits laitiers, des produits camés, des pâtes à tartiner, des substituts de repas, des boissons nutritionnelles, des glaces, des soupes et des sauces, des formules nutritionnelles cliniques et de régime.

6. Produit alimentaire selon l'une quelconque des revendications 1-5, dans lequel le produit alimentaire contient au moins 10 mg/kg de vitamine B6

7. Produit alimentaire selon l'une quelconque des revendications 1-6, dans lequel le produit alimentaire contient de la bétaine [Bet] et de la méthionine [Meth] dans un rapport molaire qui satisfait l'équation suivante : [Bet]/[Meth] ≥ 1 :1

8. Produit alimentaire selon l'une quelconque des revendications 1-7, destiné à être utilisé de traitement ou de prévention des concentrations élevées en homocystéine dans le plasma.

9. Procédé de modification d'un produit alimentaire riche en méthionine qui contient de la méthionine et optionnellement de la cystéine dans un rapport molaire qui satisfait l'équation suivante: [Cys]/[Meth] ≤ 1 :1, dans lequel le procédé comprend l'incorporation d'une source de cystéine dans une quantité suffisante pour augmenter ledit rapport à plus de 2 : 1, de préférence plus de 4 : 1.

10. Procédé selon la revendication 9, dans lequel le produit alimentaire contient additionnellement de la sérine, dans un rapport molaire qui satisfait l'équation suivante : [Ser]/[Meth] < 3 : 1, dans lequel le procédé comprend l'incorporation d'une source de sérine dans une quantité suffisante pour augmenter ledit rapport à plus de 4 : 1, de préférence plus de 6 :1.

11. Procédé selon la revendication 10 ou 9 , dans lequel la source de cystéine contient de la cystéine et optionnellement de la méthionine dans le rapport molaire suivant : [Ser]/[Meth] ≥5 : 1.

12. Procédé selon l'une quelconque des revendications 9-11 , dans lequel la source de cystéine contient de la cystéine et optionnellement de la méthionine dans le rapport molaire suivant : [Ser]/[Meth] ≥ 8 :1.

13. Utilisation de la combinaison de cystéine et de sérine pour la fabrication d'un produit destiné à être utilisé dans le traitement ou la prévention de l'hyperhomocystéinémie, ledit produit étant sélectionné dans le groupe des produits alimentaires, produits nutritionnels et préparations pharmaceutiques.
